(19) Europäisches Patentamt
European Patent Office
Office européen des brevets

(11) Veröffentlichungsnummer: **0 343 303**
**A1**

(12) **EUROPÄISCHE PATENTANMELDUNG**

(21) Anmeldenummer: **88401304.6**

(22) Anmeldetag: **27.05.88**

(51) Int. Cl.$^{4}$: **C07D 339/04 , C07D 409/04 , A61K 31/385**

(43) Veröffentlichungstag der Anmeldung: **29.11.89 Patentblatt 89/48**

(84) Benannte Vertragsstaaten: **DE FR**

(71) Anmelder: **Kali-Chemie Pharma GmbH**
**Hans-Böckler-Allee 20 Postfach 220**
**D-3000 Hannover 1(DE)**

(72) Erfinder: **Biard, Dominique**
**"Beybleu" Channeins**
**F-01190 St. Trivier sur Moignans(FR)**
Erfinder: **Christen, Marie Odile**
**27, avenue Marceau**
**F-75016 Paris(FR)**
Erfinder: **Dansette, Patrick**
**7, rue Beccaria**
**F-75012 Paris(FR)**
Erfinder: **Jasserand, Daniel**
**2, allée d'Andrezieux**
**F-75018 Paris(FR)**
Erfinder: **Mansuy, Daniel**
**192, rue de Vaugirard**
**F-75015 Paris(FR)**
Erfinder: **Sassi, Amor**
**9 Fahrat Hachal**
**Beni Hassen par Monastir(TN)**

(74) Vertreter: **Bérogin, Francis et al**
**c/o CABINET HARLE & PHELIP 21 rue de la Rochefoucauld**
**F-75009 Paris(FR)**

(54) **Neue 1,2-Dithiol-3-thion-S-oxid-Verbindungen enthaltende Arzneimittel.**

(57) Es werden die Verwendung von gegebenenfalls im Phenylring substituierten 5-Phenyl-3H-1,2-dithiol-3-thion-S-oxiden als Wirkstoffe in hepatoprotektiven Arzneimitteln und neue im Phenylring substituierte 5-Phenyl-3H-1,2-dithiol-3-thion-S-oxide beschrieben.

EP 0 343 303 A1

Xerox Copy Centre

## Neue 1,2-Dithiol-3-thion-S-oxid-Verbindungen enthaltende Arzneimittel

Die vorliegende Erfindung betrifft die Verwendung von 5-Phenyl-1,2-dithiol-3-thion-S-oxid-Verbindungen als pharmakologische Wirkstoffe, insbesondere zur Behandlung und Prophylaxe von Leberschädigungen bei größeren Säugetieren, insbesondere Menschen, und Arzneimittel, welche als Wirkstoffe 5-Phenyl-1,2-dithiol-3-thion-S-oxid-Verbindungen enthalten, sowie neue 1,2-Dithiol-3-thion-S-oxid-Verbindungen mit wertvollen pharmakologischen Eigenschaften, insbesondere hepatoprotektiven Eigenschaften.

Aus Studien über Oxidationsreaktionen von Trithionen von Perex et al (Liebigs Ann. Chem. 1981, 1510-1512), Tamayaki et al (Chem. Lett. 1980, 619-620) und Behringer et al (Phosphorus and Sulfur, 12 (1981), 115-122) ist das 5-Phenyl-3H-1,2-dithiol-3-thion-S-oxid bekannt. Für diese Verbindung ist jedoch bisher keine pharmakologische Wirksamkeit beschrieben worden.

Anetholtrithion (= 5-(4-Methoxyphenyl)-3H-1,2-dithiol-3-thion ist ein als Choleretieum im Handel befindliches Arzneimittel (Handelsprodukte Sulfarlem®, Felvitin®), von welchem bekannt ist, daß es auch hepatoprotektive Eigenschaften besitzt.

Der vorliegenden Erfindung liegt die Aufgabe zugrunde, neue Arzneimittel zur Prophylaxe und Behandlung von Leberschädigungen zu entwickeln. Ferner liegt der Erfindung die Aufgabe zugrunde, neue 1,2-Dithiol-3-thion-Derivate mit wertvollen pharmakologischen Eigenschaften herzustellen.

Es wurde nun gefunden, daß die 1,2-Dithiol-3-thion-S-oxid-Verbindungen der allgemeinen Formel I

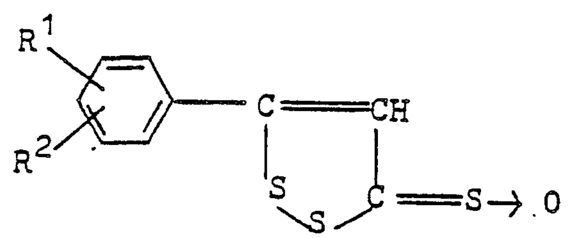

I

worin
$R^1$ Wasserstoff, Alkyl mit 1-2 Kohlenstoffatomen, niederes Alkoxy, Hydroxy, Halogen, Trifluormethyl oder Nitro bedeutet, und
$R^2$ Wasserstoff, Halogen oder niederes Alkoxy bedeutet, oder
$R^1$ und $R^2$ an benachbarte Kohlenstoffatome gebunden sind und zusammen Alkylendioxy mit 1-2 Kohlenstoffatomen bedeuten,
wertvolle pharmakologische Eigenschaften besitzen und insbesondere hepatoprotektive Eigenschaften entfalten.

Sie zeichnen sich insbesondere durch die Leber vor schädigenden Einflüssen von exogenen hepatotoxischen Substanzen schützende Wirkungen bei guter Verträglichkeit und geringer Toxizität aus.

Aufgrund ihrer pharmakologischen Eigenschaften, insbesondere ihrer hepatoprotektiven Wirkung, eignen sich die 1,2-Dithiol-3-thion-S-oxid-Verbindung der Formel I als Arzneimittel, insbesondere zur Prophylaxe und Behandlung von Leberschädigungen, beispielsweise durch hepatotoxische Dosen von Arzneimitteln, chemischen Giften oder Bestrahlungen verursachten Leberschädigungen.

Die in den Resten $R^1$ und $R^2$ der Verbindungen der Formel I enthaltenen niederen Alkylgruppen können gerade oder verzweigt sein und enthalten vorzugsweise 1-4, insbesondere 1-2 Kohlenstoffatome. Niedere Alkoxygruppen $R^1$ oder $R^2$ stellen insbesondere Methoxy dar. Als Halogensubstituenten $R^1$ oder $R^2$ kommen Fluor, Chlor oder Brom, bevorzugt Chlor oder Fluor in Frage.

Sofern $R^1$ eine Alkylgruppe bedeutet, stellt diese vorzugsweise Methyl dar und ist bevorzugt in 2- oder 3-Stellung angeordnet. Halogensubstituenten $R^1$ sind vorzugsweise in 4-Stellung angeordnet. Vorzugsweise bedeutet $R^2$ Wasserstoff.

Die 5-Phenyl-1,2-dithiol-3-thion-S-oxid-Verbindungen der Formel I weisen wertvolle pharmakologische Eigenschaften, insbesondere hepatoprotektive Eigenschaften auf und zeichnen sich durch eine gute Verträglichkeit und geringe Toxizität aus.

So besitzen die Substanzen die Fähigkeit, die Leber vor schädigenden Einflüssen, z.B. vor nach Einnahme von hepatotoxischen exogenen Substanzen auftretenden Leberschädigungen zu schützen bzw. diesen entgegenzuwirken.

Bekanntermaßen können eine Reihe von chemischen Substanzen und auch von Arzneimitteln bei entsprechend hoher Dosierung Schädigungen von Leberzellen hervorrufen und zu hepatischer Cytolyse und Lebernekrose führen. Bei cytolytischen Leberschädigungen findet eine erhöhte Freisetzung einiger Transaminasen aus den geschädigten Leberzellen in die Blutbahn statt. Bei Leberschädigungen sind daher die Blutspiegelwerte dieser Transaminasen erhöht, und die Mes sung der Serumaktivität dieser Transaminasen gibt eine Möglichkeit, Leberschädigungen festzustellen und deren Ausmaß abzuschätzen.

Eine bekannte leberschädigend wirkende Substanz ist z.B. Acetaminophen [ = Paracetamol = 4-(N-Acetylamino)-phenol ], welches in physiologisch verträglichen Dosen als Schmerzmittel allgemein verwendet wird, in hohen Dosen jedoch eine stark hepatotoxische Substanz ist. Hohe dosen von Acetaminophen führen auch in Nagetieren zu humanen Lebernekrosen vergleichbaren Leberschädigungen. Acetaminophen wird daher als hepatotoxische Standardsubstanz in pharmakologischen Standardtests in Tieren verwendet, um hepatoprotektive Eigenschaften von Testsubstanzen zu beurteilen.

Die Verbindungen der Formel I besitzen eine ausgeprägte Schutzwirkung gegenüber den leberschädigenden Wirkungen von Acetaminophen, wie sich in den nachfolgend beschriebenen pharmakologischen Standardtierversuchen zeigen läßt.

Der Metabolismus und die hepatotoxische Wirkung von Acetaminophen sind in der Literatur eingehend beschrieben worden. So ist bekannt, daß Konjugation von Acetaminophenmetaboliten an Gluthathion eine wesentliche Rolle bei der Entgiftung und Elimination von Acetaminophen spielt, und daß dessen hepatotoxische Effekte besonders stark in Erscheinung treten, wenn z.B. durch Überdosierung verursacht, Gluthathionmangel in der Zelle herrscht. Es ist ferner bekannt, daß die Hepatotoxizität zum Teil durch covalente Bindung eines metabolisch gebildeten reaktiven Agens an Makromoleküle des Lebergewebes bewirkt wird und daß Peroxidation von Lipiden der Zellmembran und dadurch bedingte Überproduktion von reaktiven Radikalen, u.a. Sauerstoffradikalen eine Schlüsselrolle bei der Hepatoxizität von Acetaminophen spielen. Die hepatoprotektiven Wirkungen der 5-Phenyl-1,2-dithiol-3-thion-S-oxid-Verbindungen der Formel I gegenüber Acetaminophen-induzierten Schädigungen können daher als Indiz dafür angesehen wer den, daß die Verbindungen die Lipidperoxidation hemmende Eigenschaften besitzen und antioxidativ und als Radikalfänger wirksam sein können.

## Beschreibung der pharmakologischen Versuche

A) Bestimmung der Schutzwirkung gegenüber der durch eine Überdosis Acetaminophen verursachten Mortalität in Mäusen.

Für die Versuche werden Gruppen von jeweils 20-30 weiblichen Mäusen mit einem Körpergewicht von ungefähr 20 g verwendet. Die Tiere erhalten während des Versuches eine tägliche Futterration, und Wasser mit pH3 steht ihnen unbegrenzt zur Verfügung.

Die Tiere werden durch i.p.-Applikation von 1000 mg/kg Acetaminophen suspendiert in 0,5 ml einer wäßrigen Gummi-arabicum-Lösung intoxiziert.

Eine Stunde vor der Intoxikation erhalten die Tiere einer Testgruppe 50 mg/kg der Testsubstanz als Suspension per os mit einer Schlundsonde appliziert. Eine Kontrollgruppe mit gleicher Anzahl Tieren erhält nur Suspensionsmittel eine Stunde vor der Intoxikation.

Die Zahl der in einer Zeitspanne von 6 Tagen verstorbenen Tiere jeder Testgruppe wird festgestellt und daraus die Schutzwirkung der Substanz in % erzieltem Schutz (= Differenz der Anzahl der in der Testgruppe verstorbenen Tiere zur Anzahl der in der jeweiligen Kontrollgruppe verstorbenen Tiere dividiert durch Anzahl der Tiere pro Gruppe) berechnet.

Die folgende Tabelle A gibt nach der vorstehend beschriebenen Testmethode erhaltene Ergebnisse wieder. Die für die Verbindungen der Formel I angegebenen Beispielsnummern beziehen sich auf die nachstehenden Herstellungsbeispiele.

Tabelle A

| Beispiel Nr. | Schutzwirkung gegenüber Acetaminophen induzierter Lethalität in Mäusen | | |
|---|---|---|---|
| | Anzahl toter Tiere/Gesamtzahl Tiere pro Testgruppe | % tote Tiere | ≙ % erzeilter Schutz |
| 11 | 1/20 | 5 | 94,6 |
| 12 | 4/20 | 20 | 78,4 |
| 4 | 1/20 | 5 | 94,6 |
| 14 | 2/20 | 10 | 89,2 |
| 6 | 3/30 | 10 | 89,2 |
| 7 | 6/30 | 20 | 78,4 |
| 8 | 14/30 | 47,6 | 49,6 |
| 9 | 3/30 | 10 | 89,2 |
| 1 | 3/30 | 10 | 89,2 |
| 5 | 9/30 | 30 | 67,6 |
| Kontrolltiergruppen | 648/700 | 92,6 | |

B) Bestimmung der Schutzwirkung gegenüber der zur Erhöhung der Transaminase-Werte im Serum führenden durch Acetaminophen induzierten Leberschädigung in Mäusen.

Für die Versuche werden weibliche Mäuse mit einem Körpergewicht von ungefähr 25 g eingesetzt. Die Tiere erhalten eine Futterration und Wasser mit pH3 steht Ihnen unbegrenzt zur Verfügung.

Die Tiere werden durch i.p.-Applikation von 450 mg/kg Acetaminophen suspendiert in 0,5 ml wäßriger Gummi-arabicum-Lösung intoxiziert.

Eine Stunde vor der Intoxikation erhalten die Tiere vorbeugend 75 mg/kg der Testsubstanz als Suspension per os mit einer Schlundsonde appliziert. Eine Gruppe von Kontrolltieren erhält nur das Suspensionsmittel eine Stunde vor der Intoxikation.

18 Stunden nach der Acetaminophen-Applikation werden die Tiere getötet und durch Durchschneiden der Halsschlagader entblutet.

Blutproben von jeweils 2 Tieren werden zusammen in ein Reagenzglas gegeben, 2-3 Stunden bei Raumtemperatur stehengelassen, eine Stunde bei 4 °C dekantiert und anschließend 10 Min. bei 2.500 x g zentrifugiert.

Das überstehende Serum wird in Plastikreagenzröhrchen überführt und darin die Aktivitäten der Transaminasen SGOT (= Serum Glutamat-Oxalacetat Transaminase) und SGPT (= Serum Glutamat-Pyruvat Transaminase) in internationalen Einheiten/1/min nach an sich bekannten Standardmethoden unter den von der deutschen Gesellschaft für Klinische Chemie empfohlenen optimierten Standardisierungsbedingungen (sieh Z klin. Chem. u. klin. Biochem. 8 (1970), 658-660 und ibid 10 (1972), 281-283) bestimmt. Die Aktivitäten werden bei einer Wellenlänge von 334 nm gemessen unter Verwendung eines automatisierten Spectrophotometers (Spectrophotometer Nr. 61 der Fa. Eppendorff) und einer Reagentienpackung für GOT und GPT Bestimmungen der Fa. Boehringer-Mannheim (= Automatenpackung GOT und GPT opt, Diagnostica Nr. 258 822 und 258 784 der Fa. Boehringer-Mannheim GmbH).

Die nach der vorstehend beschriebenen Testmethode erhaltenen Ergebnisse werden in Tabelle B wiedergegeben. In der Tabelle werden die Transaminaseaktivitäten in internationalken Einheiten/1/min angegeben. Es werden bei den nicht vorbehandelten Kontrolltiergruppen und bei den vorbehandelten Testtiergruppen jeweils die für die Gruppe aus den Einzelergebnissen gebildeten Mittelwerte (= m) und die Standardabweichungen (= sem) angegeben. Außerdem wird die Schutzwirkung in % erzielter Schutz (= Differenz der Serumaktivitätsmittelwerte zwischen Kontrollgruppe und vorbehandelter Testtiergruppe dividiert durch den Mittelwert der Kontrolltiergruppe) berechnet und angegeben.

Tabelle B

| Bsp. Nr. | Acetaminophen-induzierte Transaminaseaktivität in nicht vorbehandelten Kontrolltiergruppen | | | Acetaminophen-induzierte Transaminaseaktivität in vorbehandelten Testtiergruppen | | | % Schutz gegenüber Acetaminophen-induzierten Transaminaseaktivität | |
|---|---|---|---|---|---|---|---|---|
| | Anzahl Tiere pro Gruppe | Aktivität in intern. Einheiten /l/min (m ± sem) | | Anzahl Tiere pro Gruppe | Aktivität in internat. Einheiten /l/min (m ± sem) | | SGOT | SGPT |
| | | SGOT | SGPT | | SGOT | SGPT | | |
| 9 | 46 | 1811±330 | 2170±434 | 17 | 200±14,5 | 57,7±3 | 88,4 | 97,3 |
| 11 | 64 | 1826±255 | 2362±348 | 16 | 210± 5 | 70 ±4 | 88,5 | 97,0 |
| 12 | 63 | 1563±224 | 1611±220 | 16 | 205± 8 | 58 ±3 | 86,9 | 96,4 |
| 14 | 64 | 1914±239 | 2192±441 | 16 | 230±12 | 58 ±5 | 88,0 | 97,3 |
| 4 | 64 | 1914±239 | 2192±441 | 16 | 171± 6 | 46 ±4 | 91,1 | 97,9 |
| 1 | 46 | 1811±330 | 2170±434 | 18 | 205± 8 | 69 ±6 | 89,7 | 96,8 |
| 5 | 54 | 1413±293 | 1414±343 | 16 | 185±10 | 53,5±4 | 88,9 | 96,2 |
| 13 | 63 | 1563±224 | 1611±220 | 16 | 204± 2 | 64 ±3 | 87,0 | 96,1 |
| 6 | 62 | 2924±273 | 3586±331 | 16 | 258±16 | 85 ±3 | 91,2 | 97,6 |
| 7 | 62 | 2924±273 | 3586±331 | 16 | 322±16 | 78 ±3 | 89,0 | 97,8 |

Auf Grund ihrer pharmakologischen Eigenschaften, insbesondere ihrer hepatoprotektiven Wirkungen sind die Verbindungen der Formel I geeignet zur Prophylaxe und Behandlung von Leberschädigungen. So eignen sich die Substanzen zur Prophylaxe und Behandlung von pathologischen Zuständen, welche mit Leberschädigungen verbunden sind, z.B. Leberzirrhosen, unfreiwillige oder versehentliche Intoxikation mit hepatotoxischen Chemikalien, z.B. Herbiziden, hepatotoxischen Überdosierungen von Arzneimitteln, Behandlung mit Arzneimitteln mit hepatotoxischen Nebenwirkungen, z.B. in der Chemotherapie des Krebses, hepatotoxischen Strahlenschädigungen, z.B. in der Bestrahlungstherapie des Krebses.

Die zu verwendenden Dosen können individuell verschieden sein und variieren naturgemäß je nach Art des zu behandelnden Zustandes, der verwendeten Substanz und der Applikationsform. Im allgemeinen eignen sich jedoch für Applikationen an größeren Säugetieren, insbesondere Menschen, Arzneiformen mit einem Wirkstoffgehalt von 5-50 mg pro Einzeldosis.

Die Erfindung betrifft ferner neue 1,2-Dithiol-3-thion-S-oxid-Verbindungen der allgemeinen Formel Ia

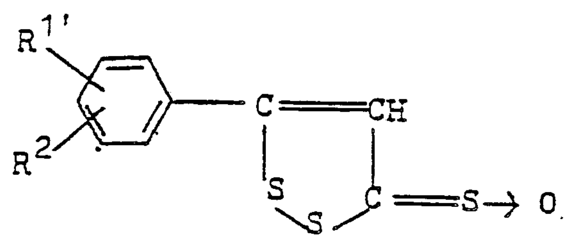

Ia

worin

$R^{1'}$ Alkyl mit 1-2 Kohlenstoffatomen, niederes Alkoxy, Hydroxy, Halogen, Trifluormethyl oder Nitro bedeutet, und

$R^2$ Wasserstoff, Halogen oder niederes Alkoxy bedeutet, oder

$R^{1'}$ und $R^2$ an benachbarte Kohlenstoffatome gebunden sind und zusammen Alkylendioxy mit 1-2 Kohlenstoffatomen bedeuten,

sowie deren Herstellung.

1,2-Dithiol-3-thion-S-oxid-Verbindungen der allgemeinen Formel I können erhalten werden, indem man entsprechende 1,2-Dithiol-3-thion-Verbindungen der allgemeinen Formel II

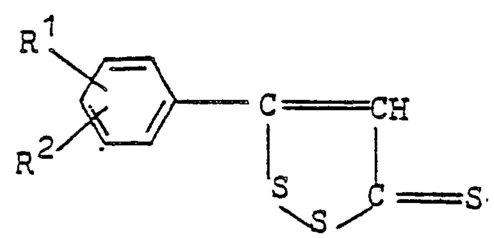

II

worin $R^1$ und $R^2$ obige Bedeutung besitzen, auf an sich bekannte Weise oxidiert.

Die Oxidation der Verbindungen der Formel II zu den entsprechenden Verbindungen der Formel I kann nach an sich zur Oxidation von 1,2-Dithiol-3-thionen zu den entsprechenden S-Oxiden bekannten Methoden, beispielsweise den in den vorstehend angeführten Arbeiten beschriebenen Methoden durchgeführt werden.

Als geeignete Oxidationsmittel nennen die vorstehend zitierten Literaturstellen beispielsweise Wasserstoffperoxid in Gegenwart eines Hydroxygruppen enthaltenden organischen Lösungsmittels, beispielsweise Essigsäure oder Methanol, Peressigsäure in einem aromatischen Kohlenwasserstoff wie Benzol, 3-Chlorperbenzoesäure in einem unter den Reaktionsbedingungen inerten aprotischen Lösungsmittel, bei spielsweise einem Halogenkohlenwasserstoff wie Dichlormethan oder Chloroform oder auch Aceton, oder Natriumperjodid in einem Gemisch aus Aceton und einem niederen Alkohol, insbesondere Methanol. Zweckmäßigerweise werden die Oxidationsmittel in etwa äquivalenten Mengen, vorzugsweise nicht mehr als 20 % Überschuß eingesetzt, und die Reaktion wird abgebrochen, sobald kein Ausgangsmaterial mehr in dem Reaktionsgemisch nachweisbar ist. Die Reaktionstemperatur kann je nach Art des verwendeten Oxidationsmittels variieren und kann z.B. zwischen -10 °C und 50 °C betragen. Gewünschtenfalls können dem Reaktionsmedium noch weitere unter den Reaktionsbedingungen inerte organische Lösungsmittel, beispielsweise aromatische Kohlenwasserstoffe, wie Benzol oder Toluol, zugesetzt werden. Als besonders günstig erweist sich die Oxidation mittels ungefähr äquivalenter Mengen (bis zu 15 % Überschuß) von 3-Chlorperbenzoesäure bei niederen Temperaturen, beispielsweise Temperaturen zwischen -25 °C und Raumtemperatur.

Die erhaltenen Verbindungen der Formel I können auf an sich bekannte Weise aus dem Reaktionsgemisch isoliert und gereinigt werden.

Die als Ausgangsprodukte verwendeten 5-Phenyl-3H-1,2-dithiol-3-thion-Verbindungen der Formel II sind bekannt und/oder können nach an sich bekannten Methoden oder analog zu an sich bekannten Methoden hergestellt werden.

So kann man zum Beispiel zur Herstellung von Verbindungen der Formel IIb

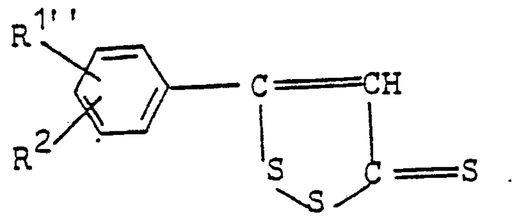

IIb

worin $R^{1''}$ die für $R^1$ angegebene Bedeutung mit Ausnahme von Hydroxy besitzt und $R^2$ obige Bedeutung besitzt, Ketoester der allgemeinen Formel III

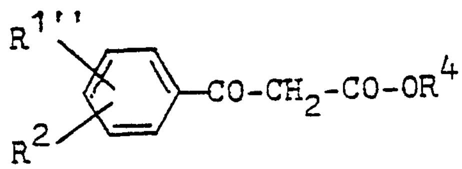

III

worin $R^{1''}$ und $R^2$ obige Bedeutung besitzen und $R^4$ niederes Alkyl, insbesondere Äthyl bedeutet, auf an sich bekannte Weise cyclisierend sulfurieren, beispielsweise durch Behandeln mit $P_4S_{10}$ oder besonders vorteilhaft nach der von Pedersen und Lawesson beschriebenen Methode (s. Tetrahedron 35, 2433-2437) durch Umsetzen mit 2,4-bis(4-Methoxyphenyl)-1,3-dithia-2,4-diphosphetan-2,4-disulfid der Formel IV

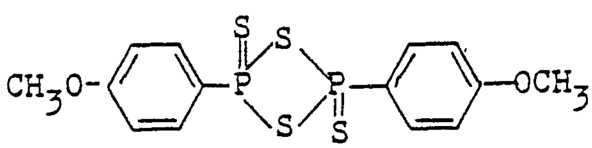

IV

(= bekannt als Lawessons Reagenz) vorzugsweise in Gegenwart von elementarem Schwefel. Die Umsetzung mit Lawessons Reagenz und elementarem Schwefel erfolgt in einem unter den Reaktionsbedingungen inerten Lösungsmittel, beispielsweise einem wasserfreien aromatischen Kohlenwasserstoff wie Toluol bei erhöhter Temperatur, zweckmäßigerweise Siedetemperatur des Reaktionsgemisches. Zweckmäßigerweise werden pro 1 Mol des Ketoesters der Formel III 1 - 2 Mole der Verbindung der Formel IV und 1 -2 Mole elementarer Schwefel eingesetzt.

Verbindungen der Formel II können auch erhalten werden, indem man Olefin-Verbindungen der Formel V

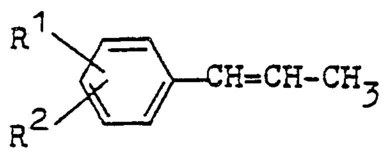

V

worin $R^1$ und $R^2$ obige Bedeutung besitzen, mit Schwefel bei erhöhter Temperatur in einem organischen Lösungsmittel umsetzt. Die Umsetzung wird vorzugsweise bei erhöhter Temperatur, beispielsweise Temperaturen von 175 - 235 °C unter Verwendung von Sulfolan als Lösungsmittel nach der in dem US-Patent Nr. 3,847,943 beschriebenen Methode durchgeführt.

Verbindungen der Formel IIb können auch ausgehend von Acetophenon-Verbindungen der Formel VI

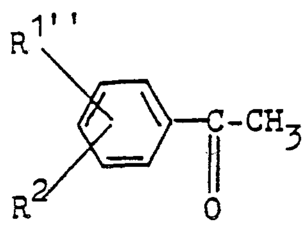

VI

worin $R^{1''}$ und $R^2$ obige Bedeutung besitzen, erhalten werden, indem man diese zunächst mit Schwefelkohlenstoff in Gegenwart einer Base zu Verbindungen der Formel VII,

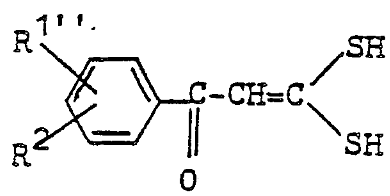

VII

worin $R^{1''}$ und $R^2$ obige Bedeutung besitzen, kondensiert und diese Zwischenprodukte weiter mit Phosphorpentasulfid zu Verbindungen der Formel II umsetzt.

Die Umsetzung von Verbindungen der Formel VI mit Schwefelkohlenstoff kann z.B. nach der von Thuillier et al beschriebenen Methode (s. Bull. Soc. Chim. France, Serie 5, Memoires presentes à la Soc. Chim. (1959), 1398-1401) durchgeführt werden. So erfolgt die Umsetzung zweckmäßig in einem inerten organischen Lösungsmittel, z.B. Benzol oder Äther unter Verwendung eines tertiären Alkalimetallalkoholates, z.B. Natriumbutylat oder -amylat. Die Umsetzung der hierbei erhaltenen Zwischenprodukte mit $P_2S_5$ erfolgt zweckmäßig in einem aromatischen Kohlenwasserstoff wie Toluol oder Xylol bei Siedetemperatur des Reaktionsgemisches.

Zur Herstellung von Verbindungen der Formel II, worin $R^1$ Hydroxy bedeutet, können entsprechende Verbindungen der Formel II, worin $R^1$ Methoxy bedeutet, in an sich bekannter Weise demethyliert werden. Beispielsweise kann die Methoxy-Gruppe durch Behandeln mit Pyridiniumchlorid sauer gespalten werden.

Die Verbindungen der Formel I können erfindungsgemäß zusammen mit üblichen pharmazeutischen Hilfs- und/oder Trägerstoffen in festen oder flüssigen pharmazeutischen Zubereitungen enthalten sein. Als Beispiele fester Präparate seien oral applizierbare Präparate wie Kapseln, Tabletten, Granulate oder Dragees genannt oder auch Suppositorien. Feste Präparate können pharmazeutisch übliche anorganische und/oder organische Trägerstoffe wie z.B. Talkum, Milchzucker oder Stärke neben pharmazeutisch üblichen Hilfsmitteln, beispielsweise Gleitmittel wie Magnesiumstearat oder Tablettensprengmitteln, enthalten. Suppositorien können an sich bekannte Suppositoriengrundlagen enthalten. Flüssige Präparate wie Lösungen, Suspensionen oder Emulsionen können die üblichen Verdünnungsmittel z.B. Wasser, oder Paraffine, und/oder Suspensionsmittel wie Polyäthylenglycole und dergleichen enthalten. Es können zusätzlich weitere Hilfsstoffe zugesetzt werden, wie z.B. Konservierungsmittel, Stabilisierungsmittel, Geschmackskorrigenzien und dergleichen.

Die Wirkstoffe können mit den pharmazeutischen Hilfs-und/oder Trägerstoffen in an sich bekannter

Weise gemischt und formuliert werden. Zur Herstellung fester Arzneiformen können die Wirkstoffe beispielsweise mit den Hilfs- und/oder Trägerstoffen in üblicher Weise gemischt und naß oder trocken granuliert werden. Je nach Art der verwendeten Zusatzstoffe kann gegebenenfalls auch durch einfaches Mischen ein direkt tablettierbares Pulver erhalten werden. Das Granulat oder Pulver kann direkt in Kapseln abgefüllt oder in üblicher Weise zu Tablettenkernen verpreßt werden. Diese können gewünschtenfalls auf an sich bekannte Weise dragiert werden.

Die nachfolgenden Beispiele erläutern die Erfindung, sollen deren Umfang jedoch in keiner Weise beschränken.

Beispiel 1

5-(4-Chlorphenyl)-1,2-dithiol-3-thion-S-oxid

a) 22,7 g (= 0,1 Mol) 4-Chlorbenzoylessigsäureäthylester, 100 g (= 0,25 Mol) Lawessons Reagenz (= 2,4-bis(4-Methoxyphenyl)-1,3-dithia-2,4-diphosphetan-2,4-disulfid) und 6,4 g (= 0,2 Mol) elementarer Schwefel werden in 100 ml wasserfreiem Toluol 10 Stunden bei 100 °C gerührt. Zur Aufarbeitung wird das das gebildete 5-(4-Chlorphenyl)-1,2-dithiol-3-thion enthaltende Reaktionsgemisch auf Raumtemperatur abgekühlt, filtriert und das Filtrat auf eine 300 g Kieselgelsäule gegeben. Das Toluol wird mit leichtem Petroläther eluiert, und dann wird das 5-(4-Chlorphenyl)-1,2-dithiol-3-thion mit einem Toluol/leichter Petroläther-Gemisch (50 : 50) eluiert. Das erhaltene Rohprodukt wird ein zweites Mal über eine Kieselgelsäule unter Verwendung von Toluol/leichter Petroläther als Elutionsmittel gereinigt und aus Toluol umkristallisiert. Es werden 9,6 g 5-(4-Chlorphenyl)-1,2-dithiol-3-thion mit einem Schmelzpunkt von 135 °C erhalten.

b) 4,9 g (= 0,02 Mol) 5-(4-Chlorphenyl)-1,2-dithiol-3-thion werden in 60 ml Chloroform gelöst. Die Lösung wird auf 0 °C abgekühlt, und zu der abgekühlten Lösung wird tropfenweise eine Lösung von 5,3 g 80-90 % reiner 3-Chlorperbenzoesäure (= ungefähr 0,026 Mol) in 150 ml Chloroform zugegeben. Nach Beendigung der etwa 30 Minuten dauernden Zugabe wird die rot gefärbte Reaktionsmischung eine weitere Stunde bei 0 °C gehalten.

Zur Aufarbeitung wird das Reaktionsgemisch zunächst mit 100 ml wäßriger gesättigter Natriumbicarbonatlösung und anschließend mit Wasser gewaschen und über Natriumsulfat getrocknet und filtriert. Die filtrierte Lösung wird unter vermindertem Druck bei Raumtemperatur auf etwa 30 ml eingeengt und auf eine 175 g Kieselgelsäule gegeben und mit Chloroform eluiert. Hierbei wird nicht umgesetztes Aus gangsmaterial zuerst abgeschieden und kann so zurückgewonnen werden, und anschließend wird das bei der Oxidation gebildete 5-(4-Chlorphenyl)-1,2-dithiol-3-thion-S-oxid erhalten. Die das S-Oxid enthaltenden Eluatfraktionen werden bei Raumtemperatur unter vermindertem Druck eingedampft. Der verbleibende Rückstand wird durch Zugabe von leichtem Petroläther kristallisiert. Das als roter Feststoff erhaltene 5-(4-Chlorphenyl)-1,2-dithiol-3-thion-S-oxid wird abfiltriert und schnell luftgetrocknet. Es werden 3,4 g des S-Oxides mit einem Schmelzpunkt von 123-125 °C erhalten.

Beispiel 2

5-(4-Hydroxyphenyl)-1,2-dithiol-3-thion-S-oxid

a) Ein Gemisch aus 100 g (= 0,41 Mol) 5-(4-Methoxyphenyl)-1,2-dithiol-3-thion und 335 g (= 2,9 Mol) Pyridiniumchlorid wird 4 Stunden auf eine Temperatur von 210-215 °C erhitzt. Anschließend wird das Reaktionsgemisch auf 80 °C abkühlen gelassen und wird dann mit 2 l warmem Wasser gewaschen, um das Pyridiniumsalz zu entfernen. Zu dem Rückstand werden 400 ml 10 %-iger wäßriger Natriumhydroxidlösung gegeben. Das ausgefallene Natriumsalz des gebildeten 5-(4-Hydroxyphenyl)-1,2-dithiol-3-thion wird abfiltriert, in 500 ml warmem Wasser gelöst, und das 5-(4-Hydroxyphenyl)-1,2-dithiol-3-thion wird durch Zugabe von Essigsäure gefällt. Der Niederschlag wird abgesaugt und getrocknet. Das erhaltene Rohprodukt wird in einem warmen Gemisch aus 300 ml Äthanol und 25 ml Diäthylamin gelöst und die Lösung auf eine kurze Aluminiumoxidsäule gegeben. Die Säule wird mit 100 ml warmem Äthanol eluiert. Die erhaltene rote Eluatlösung wird unter vermindertem Druck auf die Hälfte seines Volumens eingeengt. Hierbei kristallisiert das Diäthylaminsalz des 5-(4-Hydroxyphenyl)-1,2-dithiol-3-thions aus. Die roten Kristalle werden abfiltriert

und in 250 ml verdünnte Salzsäure gegeben. Die hierbei erhaltene Suspension wird eine Stunde bei Raumtemperatur gerührt. Sodann wird das als fester Niederschlag abgeschiedene 5-(4-Hydroxyphenyl)-1,2-dithiol-3-on abfiltriert, mit Wasser gewaschen und über Phosphorpentoxid getrocknet. Es werden 23,5 g 5-(4-Hydroxyphenyl)-1,2-dithiol-3-thion mit einem Schmelzpunkt von 192 °C erhalten.

b) Zu einer Lösung von 10 g (= 0,004 Mol) 5-(4-Hydroxyphenyl)-1,2-dithiol-3-thion in 300 ml Aceton wird unter starkem Rühren eine Lösung von 9,4 g 80-90 % reiner 3-Chlorperbenzoesäure (= ungefähr 0,046 Mol) in 200 ml Aceton innerhalb einer Stunde bei Raumtemperatur zugetropft. Anschließend wird das Reaktionsgemisch für eine weitere Stunde auf gleicher Temperatur gehalten. Das als roter Niederschlag ausgefallene 5-(4-Hydroxyphenyl)-1,2-dithiol-3-thion-S-oxid wird abgesaugt und über Phosphorpentoxid getrocknet. Es werden 9,4 g des S-Oxids mit einem Schmelzpunkt von 118-124 °C erhalten.

Nach den in den vorstehenden Beispielen beschriebenen Verfahren können auch die in der nachfolgenden Tabelle aufgeführten Verbindungen der Formel I durch Oxidation entsprechender Verbindungen der Formel II hergestellt werden.

Tabelle 1

| Beispiel Nr. | $R^1$ | $R^2$ | Fp in °C |
|---|---|---|---|
| 3 | 4-$OC_2H_5$ | H | Z |
| 4 | H | H | 110 |
| 5 | 2-Cl | 4-Cl | 129-131 |
| 6 | 2-$OCH_3$ | H | 103-104 |
| 7 | 2-F | H | 85 |
| 8 | 2-Cl | H | 82 |
| 9 | 4-$OCH_3$ | H | 91- 93 |
| 10 | 2-$NO_2$ | 4-Cl | 108-109 |
| 11 | 3-$OCH_3$ | H | 120-122 (Z) |
| 12 | 2-$OC_2H_5$ | H | Z |
| 13 | 4-$CH_3$ | H | 97 |
| 14 | 4-F | H | 97 |
| 15 | 3-$CF_3$ | H | 95- 96 |
| 16 | 3-Br | 4-$OCH_3$ | 107-110 |
| 17 | 4-OH | 3-$OCH_3$ | 184-185 |
| 18 | 3-OH | H | 182 |
| 19 | 3,4-0-$CH_2$-O | | 162 |
| 20 | 2-$CH_3$ | H | 102-104 |
| Z = Zersetzung | | | |

Beispiel I:

5-(4-Chlorphenyl)-3H-1,2-dithiol-3-thion-S-oxid enthaltende Tabletten.

Man stellt Tabletten in folgenden Zusammensetzung pro Tablette her:

| | |
|---|---|
| 5-(4-Chlorphenyl)-3H-1,2-dithiol-3-thion-S-oxid | 15 mg |
| Maisstärke | 65 mg |
| Milchzucker | 135 mg |
| Gelatine (als 10-%ige Lösung) | 6 mg |

Der Wirkstoff, die Maisstärke und der Milchzucker werden mit der 10%-igen Gelatine-Lösung eingedickt. Der Paste wird zerkleinert und das entstandene Granulat wird auf ein geeignetes Blech gebracht und bei 45 °C getrocknet. Das getrocknete Granulat wird durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke und sodann zu Tabletten von 240 mg verpreßt | 9 mg |

Beispiel II:

5-Phenyl-3H-1,2-dithiol-3-thion-S-oxid enthaltende Kapseln.

Es werden Kapseln mit folgender Zusammensetzung pro Kapsel hergestellt:

| | |
|---|---|
| 5-Phenyl-3H-1,2-dithiol-3-thion-S-oxid | 10 mg |
| Lactose | 65 mg |
| Maisstärke | 40 mg |
| lösliche Stärke | 4 mg |
| Magnesiumstearat | 1 mg |

Der Wirkstoff wird mit Lactose und Maisstärke vermischt. Die entstandene Mischung wird mit einer 15%-igen wäßrigen Lösung der löslichen Stärke durchfeuchtet und granuliert. Die feuchte Masse wird durch ein 1,6 mm-Sieb passiert, bei 40 °C getrocknet und anschließend durch ein 1,0 mm-Sieb passiert, Nach dem Vermischen des Granulats mit Magnesiumstearat wird die entstandene Mischung in Mengen von 120 mg in Kapseln abgefüllt.

Nach der in Beispiel II beschriebenen Methode werden auch Kapseln hergestellt, welche als Wirkstoff 5-(2-Methoxyphenyl)-3H-1,2-dithiol-3-thion-S-oxid oder 5-(4-Fluorphenyl)-3H-1,2-dithiol-3-thion-S-oxid enthalten.

## Ansprüche

1. 1,2-Dithiol-3-thion-S-oxid-Verbindungen der allgemeinen Formel I

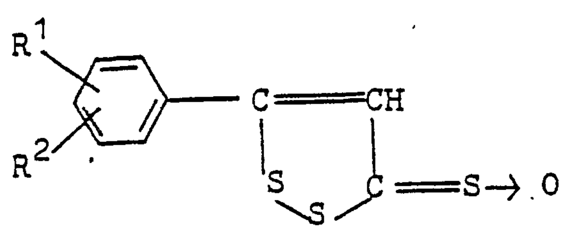

I

worin
$R^1$ Wasserstoff, Alkyl mit 1-2 Kohlenstoffatomen, niederes Alkoxy, Hydroxy, Halogen, Trifluormethyl oder Nitro bedeutet, und
$R^2$ Wasserstoff, Halogen oder niederes Alkoxy bedeutet oder
$R^1$ und $R^2$ an benachbarte Kohlenstoffatome gebunden sind und zusammen Alkylendioxy mit 1-2 Kohlenstoffatomen bedeuten,
zur Anwendung als Pharmazeutica.

2. Arzneimittel, dadurch gekennzeichnet, daß sie eine pharmakologisch wirksame Menge einer 1,2-Dithiol-3-thion-S-oxid-Verbindung gemäß Anspruch 1 und übliche pharmazeutische Hilfs- und/oder Trägerstoffe enthalten.

3. Verwendung von 1,2-Dithiol-3-thion-S-oxid-Verbindungen gemäß Anspruch 1 zur Herstellung von hepatoprotektiv wirksamen Arzneimitteln.

4. 1,2-Dithiol-3-thion-S-oxid-Verbindungen der allgemeinen Formel Ia

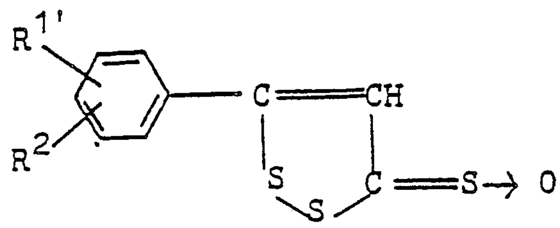

Ia

worin
$R^{1'}$ Alkyl mit 1-2 Kohlenstoffatomen, niederes Alkoxy, Hydroxy, Halogen, Trifluormethyl oder Nitro bedeutet, und
$R^2$ Wasserstoff, Halogen oder niederes Alkoxy bedeutet, oder
$R^{1'}$ und $R^2$ an benachbarte Kohlenstoffatome gebunden sind und zusammen Alkylendioxy mit 1-2 Kohlenstoffatomen bedeuten.

5. Verfahren zur Herstellung von 1,2-Dithiol-3-thion-S-oxid-Verbindungen der allgemeinen Formel Ia

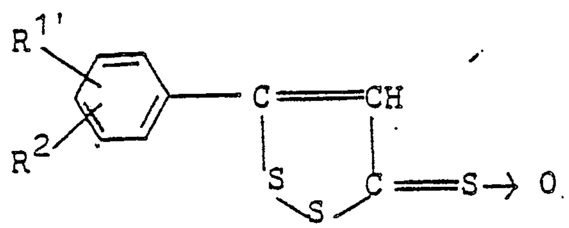

Ia

worin
$R^{1'}$ Alkyl mit 1-2 Kohlenstoffatomen, niederes Alkoxy, Hydroxy, Halogen, Trifluormethyl oder Nitro bedeutet, und
$R^2$ Wasserstoff, Halogen oder niederes Alkoxy bedeutet, oder
$R^{1'}$ und $R^2$ an benachbarte Kohlenstoffatome gebunden sind und zusammen Alkylendioxy mit 1-2 Kohlenstoffatomen bedeuten,
dadurch gekennzeichnet, daß man 1,2-Dithiol-3-thion-Verbindungen der allgemeinen Formel IIa

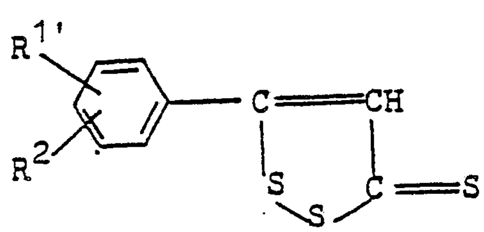

IIa

worin $R^{1'}$ und $R^2$ obige Bedeutung besitzen, oxidiert.

6. Verfahren zur Herstellung von Arzneimitteln mit hepatoprotektiven Eigenschaften, dadurch gekennzeichnet, daß man eine hepatoprotektiv wirksame Menge von 1,2-Dithiol-3-thion-S-oxid-Verbindungen gemäß Anspruch 1 zusammen mit üblichen pharmazeutischen Hilfs- und/oder Trägerstoffen in eine geeignete Arzneiform überführt.

Europäisches Patentamt

# EUROPÄISCHER RECHERCHENBERICHT

Nummer der Anmeldung

EP 88 40 1304

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,Y | CHEMICAL ABSTRACTS, Band 96, 1982, Seite 744, Zusammenfassung Nr. 162569b, Columbus, Ohio, US; H. BEHRINGER et al.: "Preparation and x-electronic structure of 1,2-dithiole-3-thione oxides (1,2-dithiol-3-ylide sulfines)", & PHOSPHORUS SULFUR 1981, 12(1), 115-22 * Zusammenfassung * | 1,4,5 | C 07 D 339/04<br>C 07 D 409/04<br>A 61 K 31/385 |
| X,Y | LIEBIGS ANN. CHEM., 1981, Seiten 1510-1512, Verlag Chemie GmbH, Weinheim, DE; M.A. PEREZ et al.: "3H-1,2-dithiol-3-thion-S-oxide" * Seiten 1510,1511 * | 1,4,5 | |
| Y | CHEMICAL ABSTRACTS, Band 89, 1978, Seite 520, Zusammenfassung Nr. 117589c, Columbus, Ohio, US; S.R. BAEHRING-KUHLMEY: "Anethole trithione", & MED. ACTUAL. 1978, 14(6), 229-32 * Zusammenfassung * | 1,4,6 | **RECHERCHIERTE SACHGEBIETE (Int. Cl.4)** |
| Y | CHEMICAL ABSTRACTS, Band 105, 1986, Seite 423, Zusammenfassung Nr. 168761j, Columbus, Ohio, US; A. NUHRICH et al.: "Heterocyclic sulfur antifungal compounds. In vitro activity of 5-aryl-1,2-dithiole-3-thiones against various fungi pathogenic for man", & ANN. PHARM. FR. 1986, 44(2), 157-62 * Zusammenfassung * | 1,4,6 | C 07 D 339/00 |
| Y | US-A-3 225 062 (E. KLINGSBERG) * Beipsiel 5 * | 4 | |
| Y | US-A-2 688 620 (O. GAUDIN) * Spalten 1,3-6 * -/- | 1,2,3,6 | |

Der vorliegende Recherchenbericht wurde für alle Patentansprüche erstellt

| Recherchenort | Abschlußdatum der Recherche | Prüfer |
|---|---|---|
| DEN HAAG | 20-01-1989 | FRANCOIS J.C.L. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 88 40 1304

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | US-A-2 556 963 (O. GAUDIN)<br>* Spalten 1-4 *<br>----- | 1,6 | |

RECHERCHIERTE SACHGEBIETE (Int. Cl.4)

Der vorliegende Recherchenbericht wurde für alle Patentansprüche erstellt

| Recherchenort | Abschlußdatum der Recherche | Prüfer |
|---|---|---|
| DEN HAAG | 20-01-1989 | FRANCOIS J.C.L. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)